(19) Europäisches Patentamt
European Patent Office
Office européen des brevets

(11) EP 2 982 760 A1

(12) EUROPEAN PATENT APPLICATION
published in accordance with Art. 153(4) EPC

(43) Date of publication:
10.02.2016 Bulletin 2016/06

(51) Int Cl.:
C12P 19/04 (2006.01)     C12P 19/44 (2006.01)
C12P 7/06 (2006.01)      C12P 7/58 (2006.01)

(21) Application number: 14829421.8

(22) Date of filing: 23.07.2014

(86) International application number:
PCT/KR2014/006712

(87) International publication number:
WO 2015/012597 (29.01.2015 Gazette 2015/04)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 23.07.2013 KR 20130086586
22.11.2013 KR 20130142834

(71) Applicant: Neo Cremar Co., Ltd.
Seognam-si, Gyeonggi-do 462-737 (KR)

(72) Inventors:
• KWON, Hyuk-Kon
Icheon-si
Gyeonggi-do 467-731 (KR)
• KIM, Na-Ri
Seongnam-si
Gyeonggi-do 462-706 (KR)
• SEO, Yi-Seul
Seoul 137-865 (KR)

(74) Representative: Tischner, Oliver
Lavoix
Bayerstrasse 83
80335 München (DE)

(54) METHOD FOR PRODUCING GALACTOOLIGOSACCHARIDE CONTAINING ENHANCED GALACTOSYLLACTOSE AS BREAST-MILK INGREDIENT

(57) The present invention relates to a method of preparing galactooligosaccharides having % a galactosyllactose content of 40 wt% or more.

Fig. 3

## Description

[Technical Field]

The present invention relates to a method of preparing galactooligosaccharides with a high content of galactosyllactose.

[Background Art]

Galactooligosaccharides are present in breast milk, and are polysaccharides having good physiological properties. Many products utilizing galactooligosaccharides have been developed (Korean Patent Publication No. 10-1997-0043065A). Particularly, galactosyllactose is known to help human immunity.

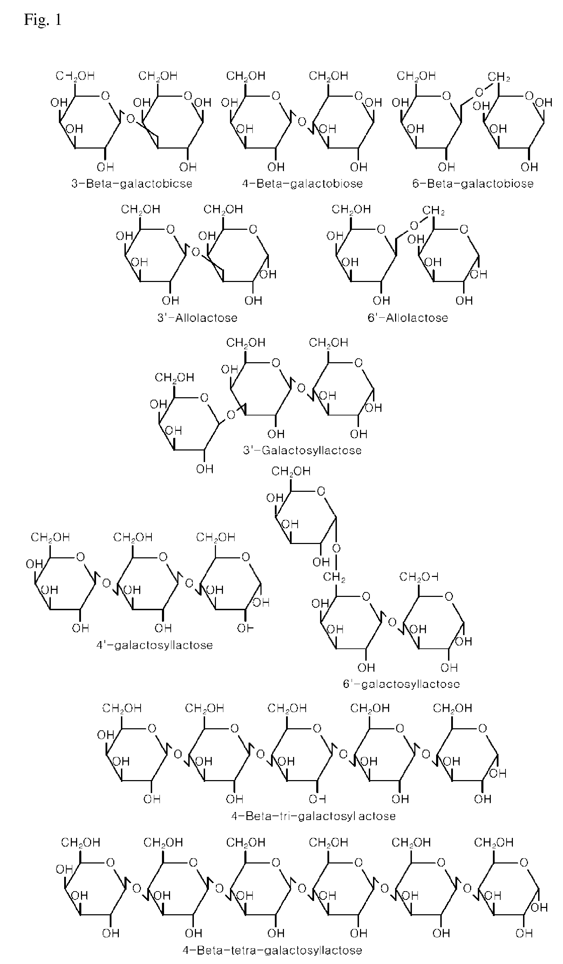

Galactosyllactose consists of trisaccharides and is a kind of galactooligosaccharide having good physiological properties prepared using lactose (see Fig. 1). Further, galactosyllactose is known to have an effect of stimulating growth of Bifidobacterium or Lactobacillus present in human large intestines, and thus is employed in foods for infants and elderly people, such as foods for improving bowel movement or diarrhea prevention, and the like. In addition, galactosyllactose is known to have an effect of inhibiting the rate of skin aging by promoting behavior of large intestine, which is assumed to be induced by smooth bowel activity through changing microflora in large intestines (an effect of stimulating growth of enteric beneficial bacteria), thereby inhibiting skin aging.

In the case of preparing galactooligosaccharides from lactose in the related art, the galactosyllactose content in galactooligosaccharides is not high. Thus, the present inventors have focused on research to find galactooligosaccharides with a high content of galactosyllactose, and confirmed that galactooligosaccharides with a high content of galactosyllactose can be prepared using specific enzymes. Based on this conformation, the present invention has been accomplished.

[Disclosure]

[Technical Problem]

It is an aspect of the present invention to provide a method of preparing galactooligosaccharides with a high content of galactosyllactose.

[Technical Solution]

In accordance with one aspect of the present invention, a method of preparing a sugar solution having a galactosyllactose content of 40% by weight (wt%) or more includes: dissolving lactose in water; adding at least one enzyme selected from 1) lactase, 2) glucose oxidase and 3) catalase, and beta-galactosidase to the lactose solution to perform enzymatic reaction; and purifying the enzyme reaction solution.

[Advantageous Effects]

The present invention is capable of preparing galactooligosaccharides having a galactosyllactose content of 40 wt% or more.

[Description of Drawings]

[0008]

　　　　Fig. 1 shows structures of galactosyllactoses.
　　　　Fig. 2 is a schematic view representing a preparation method according to the present invention.
　　　　Fig. 3 is a schematic view representing a typical method of preparing a sugar solution.

[Best Mode]

The present invention is directed to a method of preparing galactooligosaccharides having a galactosyllactose content of 40 wt% or more, including: dissolving lactose in water; adding beta-galactosidase and at least one enzyme selected from 1) lactase, 2) glucose oxidase and 3) catalase, to the lactose solution to perform enzymatic reaction; and purifying the enzyme reaction solution.

In addition, the present invention is directed to a method of preparing galactooligosaccharides having a galactosyllactose content of 40 wt% or more, including: dissolving lactose in water; adding lactase and beta-galactosidase to the lactose solution to perform enzymatic reaction; further adding yeast to the enzyme reaction solution; and purifying the enzyme reaction solution.

Further, the present invention is directed to a method of preparing galactooligosaccharides having a galactosyllactose content of 40 wt% or more, including: dissolving lactose in water; adding glucose oxidase, catalase and beta-galactosidase to the lactose solution to perform enzymatic reaction; and purifying the enzyme reaction solution.

[Mode for Invention]

Hereinafter, the present invention will be described in detail.

Lactose

Lactose to be used in the present invention may be commercially available or be directly obtained from bovine milk and the like. Further, lactose used in the present invention may be obtained by separation from by-products generated from milk processing procedures.

Galactooligosaccharide

Galactooligosaccharides to be used in the present invention are galactose-containing oligosaccharides of disaccharides to hexasaccharides prepared by enzymatic reaction and the like using lactose as a raw material. Here, lactose is excluded. Examples of galactooligosaccharides to be used in the present invention may include 4-beta-galactobiose, 6-beta-galactobiose, 3'-galactosyllactose, 4'-galactosyllactose, 6'-galactosyllactose, 4-beta-di-galactosyllactose, 4-beta-tri-galactosyllactose, 4-beta-tetra-galactosyllactose, and the like.

<Preparative Example 1>

Lactose was introduced into a reaction tank, followed by adding hot water, thereby actuating a stirrer until the concentration of the lactose solution was in the range of about 40% to about 45%. When the concentration of the lactose solution fell in the range of 40% to 45%, the temperature of the reaction tank was adjusted such that the reaction temperature of the enzyme became 55°C to 60°C. After the temperature of the reaction tank was adjusted, lactase and beta-galactosidase were introduced into the reaction tank to cause enzymatic reaction. At this time, the beta-galactosidase synthesized galactooligosaccharides from lactose and the lactase specifically decomposed lactose components.

Enzymatic reaction was allowed to proceed for about 24 hours to about 48 hours. The sugar profile of the reaction solution was analyzed using HPLC. When the content of galactooligosaccharide was 50 wt% or more, yeast was added to the reaction tank, thereby fermenting and converting the glucose and lactose components present in the sugar solution to ethanol and acetic acid.

As the glucose and lactose components were subjected to fermentation, the amount of the galactosyllactose component in galactooligosaccharides gradually and relatively increased. The galactosyllactose content in galactooligosaccharides prior to yeast fermentation was about 20%. After fermentation, the galactosyllactose content in galactooligosaccharides increased to 40 wt% or more, preferably 45 wt% or more, more preferably 50 wt% or more, even more preferably 55 wt% or more. The galactooligosaccharide content in the sugar solution increased from an initial 40% to about 75% or more. Fermentation took about 24 hours to about 48 hours. When enzymatic reaction and yeast reaction were completed, powder activated carbon was introduced into the reaction tank, followed by stirring at 70°C to 80°C for 20 minutes to 50 minutes, thereby allowing the coloring materials of the sugar solution to be adsorbed.

The sugar solution was then filtered to remove the activated carbon and foreign substances from the sugar solution, followed by removing acetic acid components through an ion purification process. The mechanism for the removal reaction is shown in Formula 1. The generated -CH$_3$COO(-) is removed by the ion purification process.

<Formula 1>     $R\text{-}OH(-) + CH_3COO(-)/H(+) \rightarrow R\text{-}CH_3COO(-) + H_2O$

Note[1] R: Resin body
Note[2] OH(-): Functional group attached to the resin body.

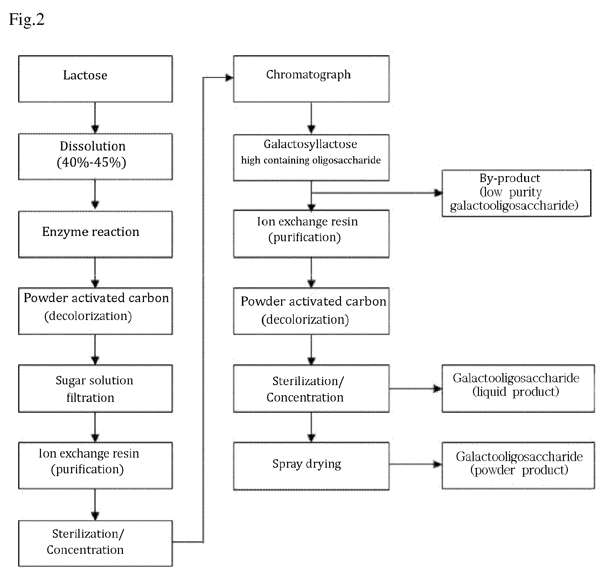

The sugar solution was subjected to ion purification to remove acetic acid, and then concentrated to a desired concentration through a concentration process. The concentration of the concentrated sugar solution may be adjusted depending upon its use and demand. Generally, the sugar solution is concentrated to a concentration of about 75% (see Fig. 2).

<Preparative Example 2>

After preparing a sugar solution in the same manner as in Preparative Example 1, beta-galactosidase, glucose oxidase and catalase were introduced into the reaction tank simultaneously or continuously to perform enzymatic reaction. For convenience of the process, the beta-galactosidase, glucose oxidase and catalase are preferably introduced simultaneously.

In the enzymatic reaction, the glucose component generated from lactose was reacted with the glucose oxidase to form gluconic acid. The reaction mechanism is shown in Formula 2. $H_2O_2$ generated in Formula 3 was removed by catalase. $R-C_6H_{12}O_6(-)$ was removed later using an ion exchange resin.

<Formula 2> 1) $C_6H_{12}O_6$ + Glucose-oxidase -> $C_6H_{12}O_7$ + $N_2O_2$ 2) $C_6H_{12}O_7$ + $H_2O_2$ + Catalase -> $H_2O_2$ + $O_2$ + $C_6H_{12}O_7$ 3) R-OH(-) + $C_6H_{12}O_7$ -> $R-C_6H_{12}O_6(-)$ + $O_3$ (Ion exchange process)

Note[1] R : Resin body
Note[2] OH(-) : Functional group attached to the resin body

The other processes after enzymatic reaction were the same as in Preparative Example 1.

Sugar solution of the present invention

The content of galactosyllactose in the sugar solution prepared by the method according to the present invention is 40 wt% or more. Preferably, the content of galactosyllactose in the sugar solution prepared by the method according to the present invention is 45 wt% or more.

Further, the content of glucose and galactose in the sugar solution prepared by the method according to the present invention is less than 20 wt%. The content of glucose and galactose of less than 20 wt% means that glucose and galactose are present in an insignificant amount or are substantially not present.

The advantages and features of the present invention, and methods for accomplishing the same will be described in more detail with reference to the following examples together with the accompanying drawings. It should be understood that the present invention may be embodied in different ways, and that the embodiments are given to provide complete disclosure of the invention and to provide thorough understanding of the invention to a person having ordinary knowledge in the art to which the present invention pertains. The present invention is only defined by the appended claims and equivalents thereof.

<Materials and methods>

As raw materials lactose and enzymes were purchased from commercially available products. As by-products obtained from manufacture of cheese from milk, whey was purchased from Maeil Dairies Co., Ltd.

HPLC analysis

Ultrapure water (specific electrical conductivity value: 0.05 μS/cm or less) was added to a sugar solution sample to dilute the sample to a concentration of about 2.5% to about 3.0%. (Analysis of sample concentration was performed using refractometer). After adjusting the concentration of the sugar solution sample, foreign substances contained in the sample solution were removed using a 0.2-0.45μm filter. The obtained sample was subjected to HPLC analysis.

The conditions for HPLC analysis were as follow:

- Column for HPLC analysis: Polyamine-based column (YMC-Pack Polyamine-/Japan)
- Column size, grain size: 250 x 4.6 mmI.D.s - 5μm, 12nm
- Detector : RID (Refractive Index Detector)
- Column oven temperature : 25°C-30°C
- Solvent: 64% Acetonitrile
- Flow rate: 1.0 mL/min
- Run time: 25 min
- Injection volume: 20μL
- Standard reagents: Glucose(Dextrose), Galactose, Lactose

First, prior to analysis of the corresponding samples, standard reagents were analyzed in the same manner as in HPLC analysis to identify the retention time of peaks depicted in chromatograms of each standard reagent. The pretreated samples for analysis were injected into HPLC columns to produce HPLC chromatograms. When the retention time of glucose, galactose and lactose as standard reagents corresponds to those of the samples, they were considered to be identical components. Then, the galactooligosaccharide content was calculated in accordance with Formula 3:

<Formula 3>

Galactooligosaccharide content (DB%) = 100 - (glucose content + galactose content + lactose content)

Note[1] DB%: Dry Basis. DB% was calculated based on anhydrides.

<Example 1>

Lactose was prepared from commercially available products. Lactose was introduced into a reaction tank, followed by adding hot water, thereby actuating a stirrer until the lactose content in 100 wt% of the lactose solution was in the range of about 40 wt% to about 45 wt%. When the concentration of the lactose solution fell in the range of 40% to 45%, the temperature of the reaction tank was adjusted such that the reaction temperature of enzymes was 55°C to 60°C. After the temperature of the reaction tank was adjusted, lactase and beta-galactosidase were introduced into the reaction tank to cause enzymatic reaction. At this time, the beta-galactosidase synthesized galactooligosaccharides from lactose and the lactase specifically decomposed lactose components.

Enzymatic reaction was allowed to proceed for 36 hours. The sugar profile of the sugar solution was analyzed using HPLC. When the galactooligosaccharide content was 50 wt% or more, yeast was introduced into the reaction tank to cause fermentation of glucose and galactose components present in the generated galactooligosaccharide, thereby converting those components into ethanol and acetic acid. When the enzymatic reaction and yeast reaction were completed, powder activated carbon was introduced into the reaction tank, followed by stirring at 75°C for about 30 minutes, thereby allowing coloring materials of the sugar solution to be adsorbed.

Subsequently, the sugar solution was filtered to remove the activated carbon and foreign substances from the sugar solution. Thereafter, acetic acid components were removed through ion purification.

After subjecting the sugar solution to ion purification to remove acetic acid, the sugar solution was concentrated such that the solid content in the sugar solution had a concentration of 75%. Namely, the sugar solid content in the concentrated sugar solution was 75 wt% and water content was 25 wt%.

<Example 2>

Lactose was prepared from commercially available products. A lactose solution was prepared in the same manner as in Example 1. The temperature of the reaction tank was adjusted such that the reaction temperature for yeast became 55°C to 60°C. Thereafter, beta-galactosidase, glucose oxidase and catalase were introduced into the reaction simultaneously tank, and then enzymatic reaction was allowed to proceed for 60 hours. The glucose component generated from lactose in the enzymatic reaction reacted with glucose oxidase to form gluconic acid.

After completing the enzymatic reaction, the coloring materials in the sugar solution were adsorbed in the same manner as in Example 1. The sugar solution was filtered and subjected to ion purification. Gluconic acid generated in the enzymatic reaction was removed by ion purification.

<Example 3>

Whey was prepared from commercially available products. Lactose was separated from whey. The separation was performed by adjusting pH of whey to pH 4.4 to pH 4.6, heating whey to about 60°C, leaving whey for about 1 hour or so to coagulate protein components, and then separating protein components and lactose components by filtration of the sugar solution. A sugar solution was prepared in the same manner as in Example 1 except that the isolated lactose was employed.

<Comparative Example 1>

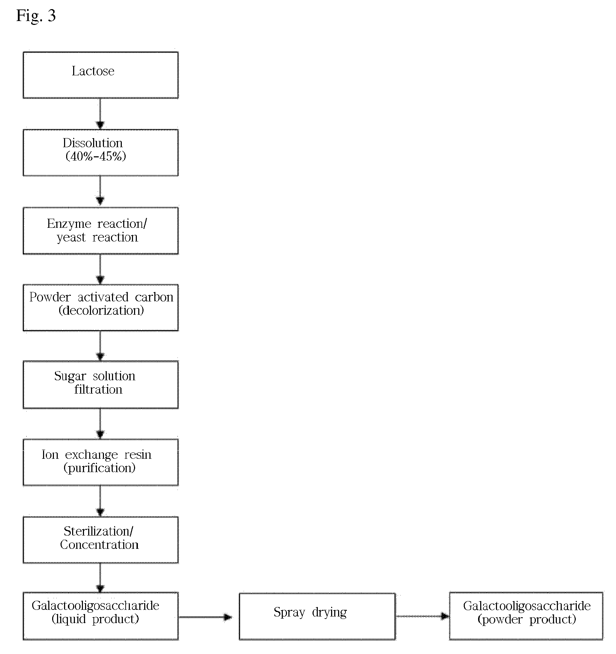

A sugar solution was prepared in accordance with the prior method. Specifically, lactose was introduced into a reaction tank, followed by adding hot water, thereby actuating a stirrer until the concentration of a solid content in the lactose solution was in the range of about 40 wt% to about 45 wt%. When the concentration of the solid content in the lactose solution fell in the range of 40 wt% to 45 wt%, beta-galactosidase was introduced into the reaction tank to perform enzymatic reaction. The enzymatic reaction was allowed to proceed for 60 hours. After completion of the enzymatic reaction, powder activated carbon was introduced into the reaction tank, followed by stirring the sugar solution at 75°C for about 30 minutes, thereby allowing the coloring materials of the sugar solution to be adsorbed. The sugar solution was then filtered to remove the activated carbon and foreign substances from the sugar solution, followed by ion purification using an ion exchange resin. The ion purified sugar solution was subjected to sterilization, concentrated to a concentration of 50%, and then subjected to chromatography. After purifying the resulting sugar solution using an ion exchange resin, powder activated carbon was introduced again to adsorb coloring materials, which were then sterilized and concentrated to a concentration of 75% (see Fig. 3).

<Experimental Example 1>

Sugar profile depending upon preparation methods

The sugar profile of the sugar solutions according to Examples 1 to 3 was analyzed. As the result, it was found that the sugar solutions of Examples 1 to 3 showed significantly high galactosyllactose content as compared to that of Comparative Example 1 (see Table 1).

TABLE 1

| Sugar profile | Comparative Example 1 | Example 1 | Example 2 | Example 3 |
|---|---|---|---|---|
| Glucose + Galactose | 30.5 | 5.0 | 5.8 | 0.0 |
| Galactobiose | 23.1 | 25.5 | 25.2 | 20.0 |
| Lactose | 22.4 | 19.3 | 18.9 | 25.0 |
| Galactosyllactose | 16.8 | 43.4 | 43.9 | 45.0 |
| Galactooligosaccharides more than tetrasaccharides | 7.2 | 6.8 | 6.2 | 10.0 |
| Total Galactooligosaccharides | 47.1 | 75.7 | 75.3 | 75.0 |
| Unit: DB% DB%: Anhydride basis (Dry Basis) *Total galactooligosaccharides (TOS): Galactobiose + Galactosyllactose + Galactooligosaccharides of tetrasaccharides or more | | | | |

<Experimental Example 2>

Difference in yield of the sugar solutions depending upon preparation methods

Yield of Examples 1 and 2 was analyzed. As a result, it was found that Examples 1 and 2 showed significantly high yield as compared to Comparative Example 1 (see Table 2).

TABLE 2

| Item | Comparative Example 1 | Novel Preparation method (Fermentation method) (Example 1) | Reference (Example 2) |
|---|---|---|---|
| Preparation type | Batch type/Continuous type | Yeast fermentation method | Enzymatic reaction |
| Preparation yield (%) (* Total oligosaccharide) | 75.4/81.9 | 86.7 | 83.7 |
| Product purity (%) (Total oligosaccharide content) | 75.0 or more | 75.0 or more | 75.0 or more |
| *Total oligosaccharide: Oligosaccharide content including galactosyllactose | | | |

[Industrial Applicability]

The present invention provides a method of preparing galactooligosaccharides with a high content of galactosyllactose.

**Claims**

1.   A method of preparing galactooligosaccharides having a galactosyllactose content of 40 wt% or more, comprising:

   dissolving lactose in water;
   adding beta-galactosidase and at least one enzyme selected from 1) lactase, 2) glucose oxidase and 3) catalase to the lactose solution to perform enzymatic reaction; and
   purifying the enzyme reaction solution.

2.   The method of preparing galactooligosaccharides according to claim 1, wherein the enzyme reaction is performed by further adding yeast after the lactase and the beta-galactosidase are added to the lactose solution.

3.   The method of preparing galactooligosaccharides according to claim 2, wherein, after adding the lactase and the beta-galactosidase to the lactose solution, yeast is further added when the galactooligosaccharide content in the lactose solution is 50 wt% or more.

4.   The method of preparing galactooligosaccharides according to claim 2, wherein glucose and galactose are converted into ethanol and acetic acid by the yeast.

5.   The method of preparing galactooligosaccharides according to claim 1, wherein the enzyme reaction is performed by adding the glucose oxidase and the catalase to the lactose solution.

6.   The method of preparing galactooligosaccharides according to claim 5, wherein the glucose oxidase and the catalase are simultaneously or continuously added to the lactose solution.

7.   The method of preparing galactooligosaccharides according to claim 5, wherein glucose is converted into gluconic acid by the glucose oxidase.

8.   The method of preparing galactooligosaccharides according to claim 5, wherein hydrogen peroxide is removed by the catalase.

9.   The method of preparing galactooligosaccharides according to claim 1, wherein the galactooligosaccharide has a galactosyllactose content of 40 wt% or more.

10.  The method of preparing galactooligosaccharides according to claim 1, wherein the enzyme reaction solution has a glucose and galactose content of less than 20 wt%.

11.  The method of preparing galactooligosaccharides according to claim 1, wherein the lactose is obtained from by-products generated in a milk processing procedure.

12.  The method of preparing galactooligosaccharides according to claim 1, wherein the lactose is derived from whey.

Fig. 1

Fig.2

Fig. 3

```
┌─────────────────────────┐
│         Lactose         │
└─────────────────────────┘
             │
             ▼
┌─────────────────────────┐
│       Dissolution       │
│       (40%-45%)         │
└─────────────────────────┘
             │
             ▼
┌─────────────────────────┐
│    Enzyme reaction/     │
│     yeast reaction      │
└─────────────────────────┘
             │
             ▼
┌─────────────────────────┐
│  Powder activated carbon│
│      (decolorization)   │
└─────────────────────────┘
             │
             ▼
┌─────────────────────────┐
│     Sugar solution      │
│        filtration       │
└─────────────────────────┘
             │
             ▼
┌─────────────────────────┐
│   Ion exchange resin    │
│      (purification)     │
└─────────────────────────┘
             │
             ▼
┌─────────────────────────┐
│     Sterilization/      │
│      Concentration      │
└─────────────────────────┘
             │
             ▼
┌─────────────────────────┐      ┌──────────────┐      ┌──────────────────────────┐
│  Galactooligosaccharide │─────▶│ Spray drying │─────▶│  Galactooligosaccharide  │
│     (liquid product)    │      │              │      │     (powder product)     │
└─────────────────────────┘      └──────────────┘      └──────────────────────────┘
```

## INTERNATIONAL SEARCH REPORT

| International application No. |
|---|
| **PCT/KR2014/006712** |

### A.  CLASSIFICATION OF SUBJECT MATTER

*C12P 19/04(2006.01)i, C12P 19/44(2006.01)i, C12P 7/06(2006.01)i, C12P 7/58(2006.01)i*

According to International Patent Classification (IPC) or to both national classification and IPC

### B.  FIELDS SEARCHED

Minimum documentation searched (classification system followed by classification symbols)
C12P 19/04; C12P 19/00; C12P 19/44; C12P 7/06; C12P 7/58

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: galactosyllactose, galacto oligosaccharide, beta-galactosidase, catalase, glucose oxidase, lactase, yeast

### C.  DOCUMENTS CONSIDERED TO BE RELEVANT

| Category* | Citation of document, with indication, where appropriate, of the relevant passages | Relevant to claim No. |
|---|---|---|
| A | KR 10-0945306 B1 (SAMYANG GENEX CORPORATION) 03 March 2010<br>See abstract and the claims. | 1-12 |
| A | ONISHI,N. et al. Galacto-oligosaccharide production from lactose by Sirobasidium magnum CBS6803.<br>Letters in Applied Microbiology. 1996, vol. 23, pp. 253-256.<br>See abstract and experimental method. | 1-12 |
| A | PETROVA,S.D. et al. Production of Galacto-oligosaccharides from Lactose and whey Permeate by beta-Glycosidase from Sporotrichum Thermophile ATCC 34628.<br>Biotechnol. Biotechnol. EQ. 2009, vol. 23, pp. 672-675.<br>See abstract and experimental method. | 1-12 |

☐  Further documents are listed in the continuation of Box C.          ☒  See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br><br>26 SEPTEMBER 2014 (26.09.2014) | Date of mailing of the international search report<br><br>**26 SEPTEMBER 2014 (26.09.2014)** |
| Name and mailing address of the ISA/**KR**<br>　　　Korean Intellectual Property Office<br>　　　Government Complex-Daejeon, 189 Seonsa-ro, Daejeon 302-701,<br>　　　Republic of Korea<br>Facsimile No.  82-42-472-7140 | Authorized officer<br><br><br>Telephone No. |

Form PCT/ISA/210 (second sheet) (July 2009)

INTERNATIONAL SEARCH REPORT
Information on patent family members

| International application No. |
| --- |
| **PCT/KR2014/006712** |

| Patent document cited in search report | Publication date | Patent family member | Publication date |
| --- | --- | --- | --- |
| KR 10-0945306 B1 | 03/03/2010 | KR 10-2008-0073759 A | 03/07/2009 |

Form PCT/ISA/210 (patent family annex) (July 2009)

EP 2 982 760 A1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1019970043065 A